# EUROPEAN PATENT APPLICATION

(11) **EP 4 814 969 A1**
(43) Date of publication of application: **30.09.2026**
(21) Application number: 25382288.6
(22) Date of filing: 26.03.2025
(51) Int. Cl.: G16B 15/00, G16B 15/20, G16B 15/30

(54) **VARIANTS IN POLYPEPTIDES**

(71) Applicant: Fundació Centre de Regulació Genòmica, 08003 Barcelona (ES); Institució Catalana De Recerca I Estudis Avançats (ICREA), 08010 Barcelona (ES)
(72) Inventor: CIANFERONI, Damiano, 08001 BARCELONA (ES); SERRANO PUBUL, Luis Felipe, 08950 ESPLUGUES DE LLOBREGAT (ES); DELGADO BLANCO, Francisco Javier, 08003 BARCELONA (ES); HAMDANI, Rahma, 08003 BARCELONA (ES)
(74) Representative: ZBM Patents - Zea, Barlocci & Markvardsen

(57) **Abstract**

There is provided a computer-implemented method of assigning an identity of an amino acid residue to a reference position in a polypeptide backbone.

## Description

### TECHNICAL FIELD

This disclosure relates to a computer-implemented method for determining polypeptide sequence variants.

### BACKGROUND

Proteins are major structural and functional elements of all living organisms. The sequence of amino acid residues in the polypeptide chain or chains forming a protein determines the protein's three-dimensional (3D) structure and, thus, a protein's function, given that the biological properties of proteins depend directly on the protein's 3D conformation. For example, the conformation determines the activity of enzymes, the capacity and specificity of binding proteins, and the structural attributes of receptor molecules. Some advancements in machine learning have allowed effective designs in generating new molecules with desired functions. Computational protein design is the task of determining amino acid sequences of desired characteristics and typically relies on first principles, also referred to as physical principles. Specifically, these are based on approximating the free energy of a given amino acid sequence through modelling of the underlying inter-atomic interactions or contacts present in the sequence of interest. Additionally, computational protein design strategies also often employ side chain and/or backbone exploration strategies. Some challenges of computational protein design methods known in the prior art may be related to the fact that the size of the sequence space, i.e. the number of possible sequence combinations, increases exponentially with the number of residues to be assigned/modified, and the well-acknowledged risk of getting trapped into local minima associated with methods that comprise assigning rotamers sequentially.

Prior art methods disclose strategies to reduce the size of the sequence space and modifications that minimise the risk of the local minima trap. However, these do not improve accuracy and the sequence diversity of polypeptide variants generated with prior art methods remains low. While further strategies have been proposed that may allow prior art methods to improve sequence diversity in the resulting variants, this is accompanied by an undesirable increase in the number of non-functional or unstable variants that fail to fold correctly or to adopt the desired three-dimensional structure. Moreover, the functionality and predicted structure of polypeptide variants designed with prior art methods have not typically been experimentally validated.

A further limitation of prior art methods lies in the fact that they handle poorly non-protein entities within the design process, which hampers their versatility and limits their scope of applicability.

There are no methods for protein design allowing the design of protein sequence variants of a desired geometry with high prediction accuracy and high sequence diversity and/or high degree of divergence from original sequence, particularly for polypeptides that may be in complex with further interaction partners.

### SUMMARY

In a first aspect, the present disclosure provides a computer-implemented method of assigning an identity of an amino acid residue to a reference position in a polypeptide backbone, the identity being determined by the amino acid residue side-chain;
the polypeptide backbone comprised within a macromolecular structure, the macromolecular structure comprising a plurality of elements, the elements comprising:
   **an amino acid backbone in the reference position or an amino acid residue in the reference position;
   **one or more neighbouring elements of a list consisting of: one or more further amino acid residues, one or more small molecules, one or more inorganic molecules, one or more nucleotides, at least a part of a lipid molecule, at least a part of a carbohydrate molecule, and combinations thereof; wherein the one or more neighbouring elements comprises at least one atom within a -given- neighbouring distance from at least one atom of the amino acid backbone in the reference position or of the amino acid residue in the reference position;
the computer-implemented method comprising:
   obtaining the reference position;
   obtaining spatial coordinates of
      at least one atom comprised in the amino acid backbone in the reference position or in the amino acid residue in the reference position; and
      at least one atom per neighbouring element;
   determining at least one reference multiplet of atoms, the reference multiplet consisting of one atom comprised in the amino acid backbone in the reference position or in the amino acid residue in the reference position and one atom of each of one or more neighbouring elements; wherein the reference multiplet of atoms is associated to a reference geometrical property; wherein the distance between the atoms of the reference multiplet comprised within each pair of two or more neighbouring elements is equal to or less than the neighbouring distance;
   iteratively, per each one of the reference multiplet of atoms, finding, in a database, at least one matching multiplet of atoms;
      wherein the database comprises
         multiplets of atoms within elements deemed to interact in empirical macromolecular structures, and,
         for each one of the multiplets:
            an identity of each element comprising each one of the atoms,
            and an associated geometrical property;
      wherein the associated geometrical property of the at least one matching multiplet of atoms substantially equals the reference geometrical property;
      wherein the atoms in each matching multiplet are ordered according to an ordered relationship between atoms;
determining the identity of the amino acid residue as the identity of an element deemed to interact comprising the atom of the at least one matching multiplet which corresponds, according to the ordered relationship, to the atom of the reference multiplet comprised in the amino acid backbone in the reference position or in the amino acid residue in the reference position;
and assigning the identity of the amino acid residue to the reference position in the polypeptide backbone.

An identity of an amino acid residue is determined by the amino acid residue side-chain.

Assigning an identity of an amino acid residue to a reference position in a polypeptide backbone comprises determining or computing, via a simulation algorithm, an amino acid residue to either replace an amino acid residue present in a reference position in a starting or original polypeptide, or, alternatively, to add a side-chain to an alpha carbon atom of an amino acid backbone present in the reference position of such starting or original polypeptide to form an amino acid residue in the reference position. The reference position is obtained which may imply determine or receive or select.

The polypeptide backbone is comprised within a macromolecular structure. The macromolecular structure comprises the amino acid backbone or the amino acid residue in the reference position, where an identity of an amino acid residue is to be assigned. In examples, where the polypeptide backbone comprises an amino acid residue in the reference position, the assigned identity of the amino acid residue may be different to the identity of the amino acid residue occupying the reference position in the starting or original polypeptide. The macromolecular structure comprises, furthermore, the so called one or more neighbouring elements. The neighbouring elements may comprise both natural and unnatural or synthetic or artificial or non-canonical elements.

Assigning an identity of an amino acid residue to a reference position in a polypeptide backbone may allow, depending on the backbone and the circumstances, to find an improved polypeptide compared to the original polypeptide in terms of, for example, stability, affinity, specificity, activity, or aggregation propensity, as will be explained herein.

The computer-implemented method, CI method, comprises obtaining the reference position in the polypeptide backbone to which the identity of the amino acid residue is to be assigned, in the macromolecular structure. The CI method further comprises obtaining spatial coordinates of at least one atom comprised in the polypeptide backbone -or one atom of the polypeptide backbone- within the reference position; and at least one atom per neighbouring element.

The reference position and the spatial coordinates obtained by the CI method may be referenced to a coordinate system, or a 3-dimensional coordinate system, in which the steps of the CI method are implemented. For example, the reference position may indicate a position in a coordinate system and the spatial coordinates may be reference to the same coordinate system.

The CI method may obtain thereby a digital model of a part of the macromolecular structure in which the identity of the amino acid residue is to be assigned. The digital model may comprise a computer-implemented data structure to represent the spatial coordinates of the at least one atom comprised in the amino acid backbone in the reference position or in the amino acid residue in the reference position and of the at least one atom per neighbouring element. In the present disclosure, each spatial coordinate (X, or Y, or Z) or a set of spatial coordinates (X, Y, Z) of the at least one atom of the elements being digitally modelled may be mapped to at least one spatial coordinate or a set of spatial coordinates stored in the CI data structure. The data structure may comprise a memory. The memory may be for example an EEPROM, or may be external, for example, data storage means such as magnetic disks, e.g., hard disks, optical disks, e.g., DVD or CD, memory cards, flash memory, e.g., pen drives, or solid-state drives, SSD based on RAM, based on flash, etc. The spatial coordinates may comprise atomic coordinates describing the position of an atom in a three-dimensional space relative to a defined reference system, e.g., a Cartesian coordinate system. Atomic coordinates represent the position of an atom within a specific reference frame, typically in three-dimensional space. In a protein or macromolecule, atomic coordinates specify the location of individual atoms, e.g., carbon, nitrogen, oxygen, relative to the molecular structure. The spatial coordinates represent therefore spatial positions of atoms in the part of the macromolecular structure such that dimensions, distances and other metrics may be calculated or computed. The data structure may store a plurality of spatial coordinates of the at least one atom comprised in the amino acid backbone in the reference position or in the amino acid residue in the reference position and of the at least one atom per neighbouring element.

The reference position may be obtained or received or retrieved formatted in an atomic coordinate data file, such as a file that specifies the positions of each atom in space, e.g., using X, Y and Z Cartesian coordinates, and the chemical element each atom represents. A variety of atomic coordinate data file formats suitable for use with the present invention are known to the person skilled in the art and include but are not limited to: PDB (Protein Data Bank) data file format, mmCIF (macromolecular crystallographic information format). The reference position may comprise an amino acid residue number of the polypeptide backbone. The coordinate data file may encode the amino acid residue number.

The spatial coordinates may be obtained or received or retrieved formatted in an atomic coordinate data file, such as a file that specifies the positions of each atom in space, e.g., using X, Y and Z Cartesian coordinates, and the chemical element each atom represents. A variety of atomic coordinate data file formats suitable for use with the present invention are known to the person skilled in the art and include but are not limited to: PDB (Protein Data Bank) data file format, mmCIF (macromolecular crystallographic information format), and ASN.1 (Abstract Syntax Notation One) data format.

The CI method further comprises determining at least one reference multiplet of atoms. The multiplet of atoms may comprise 2 or 3 or 4 or 5 or more atoms in, respectively, a pair, or a triplet, or a quartet, or a quintet. The reference multiplet consists of one atom comprised in the amino acid backbone occupying the reference position within the polypeptide backbone, or in the amino acid residue occupying the reference position within the polypeptide backbone, and one atom of each of one or more neighbouring elements. The reference multiplet of atoms is associated to a reference geometrical property. A geometrical property may, for example, be a multiplet of distances between atoms in the reference multiplet. In an illustrative example, if the reference multiplet is a reference triplet of atoms [A, B, C], then the associated geometrical property may comprise a triplet comprising distances between atoms A, B, C, for example the geometrical property being [dA-B, dA-C, dB-C], where for example dA-B is the distance between the atom A to the atom B. The reference multiplet comprises atoms which comply with a limitation such that the distance between the atoms of the reference multiplet comprised within each pair of two or more neighbouring elements is equal to or less than a neighbouring distance. In other words, and following the illustrative example, the atom A may belong to the polypeptide backbone in the reference position, and the atoms B and C may belong to different neighbouring elements, and the three atoms comply with the limitation that dA-B, the distance between A and B, dB-C, the distance between B and C, and dA-C, the distance between A and C, are all equal to or less than a neighbouring distance. The neighbouring distance may, for example, be the largest distance observed in an existing database between corresponding atoms; for example, in the case of a Cα - Cα distance, the largest distance may be comprised in a range of 16 Å (Angstroms) to 19 Å, for example 18 Å. In examples, the atoms are the carbon-α atoms in different amino acid residues and the neighbouring distance is equal or less than 18 Å. The neighbouring distance may be, in examples, a distance allowing one atom of an amino acid residue or an amino acid backbone in the reference position to interact or contact with one atom of a neighbouring element, e.g., a neighbouring amino acid residue. In other words, the distance between two atoms of two respective neighbouring elements that comprise atoms comprised in the reference multiplet is equal to or less than the neighbouring distance. In the CI methods of the present disclosure, distances between atoms are calculated based on their spatial coordinates. The CI method may comprise determining at least one (i.e., one, two, or more) reference multiplets.

Once the at least one reference multiplet is determined, the CI method further comprises: iteratively, per each one of the determined reference multiplet of atoms, finding, in a database, at least one matching multiplet of atoms. The matching multiplet may comprise a multiplet that geometrically matches the reference multiplet. In other words, the associated geometrical property of the at least one matching multiplet of atoms substantially equals the reference geometrical property. In the present disclosure, the fact that the associated geometrical property substantially equals the reference geometrical property comprises that both geometrical properties are equal with a tolerance, where the tolerance may for example be in range from 0 % to 20 % of the greatest geometrical property, or may be between 0 Å and 3 Å. The tolerance may be predefined by, for example, a user, or may be a predefined parameter in the CI method.

The geometrical property may comprise a multiplet of distances between pairs of atoms in the multiplet. The geometrical property may comprise a multiplet of angles between lines joining pairs of atoms in the multiplet. The geometrical property may comprise angles between lines joining pairs of atoms in the multiplet. The geometrical property may comprise dihedrals between planes comprising three or more of the atoms in the multiplet. The geometrical property may comprise one or more angles between a line joining a pair of atoms in the multiplet and a plane comprising three or more of the atoms in the multiplet.

In the case of a triplet, the geometrical property may geometrically represent 3 edges of a triangle. In the case of a quartet, the geometrical property may geometrically represent 4 edges of a quadrangle, or may represent lines joining all atoms with the rest of atoms in a quadrangle. The tolerance may be varied between 0% to 100% of a geometrical property, for example the tolerance may varied between -20% to +20%. As an illustrative example, considering the case where the geometrical property comprises distances between atoms of the multiplet, one of the distances of the geometrical property may be allowed to be a 100% of the distance, in other words the tolerance for the geometrical property may be 100%, so that a multiplet comprising n components, may be matched with a multiplet having fewer components,. In an example of a triplet, the triplet may in fact be matched with a pair, where a pair is understood as a triplet forming a triangle where, for one of the distances between a pair of atoms, the tolerance is 100% so that a comparison between the geometrical properties being "distance between atoms" with a "non-existing" third atom is 100%. In such a case, a triangle may be matched with a line. In an example of a quartet, the quartet may in fact be matched with a pair. In an example of a quartet, the quartet may alternatively be matched with a triplet. The tolerance being 100% may be advantageous in cases where, for example, a match with a multiplet containing all the components is not being found for a particular reference position and for specific design requirements. In an example of a triplet, the triplet may be matched with a pair, where a pair is understood as a matching triplet where the tolerance with one of the distances between a pair of atoms is infinite In such a case, a triangle may be matched with a line.

The atoms in each multiplet, e.g., reference multiplet or matching multiplet, are ordered according to an ordered relationship between atoms, such that the associated geometrical property substantially equals the reference geometrical property in a specific order or position in space, as will be illustrated by the detailed description and figures of the present disclosure.

The database comprises multiplets of atoms, wherein each atom in a multiplet is comprised within a different element, wherein the elements that comprise the atoms of the multiplet are deemed to interact in empirical macromolecular structures. Such empirical macromolecular structures may comprise naturally-occurring and non-natural macromolecular structures or digitally- or virtually-generated macromolecular structures. In the context of the database of the present disclosure, the elements comprising the atoms of a multiplet are "deemed to interact" if two limiting conditions are met: (i) each element comprises at least one atom - wherein the at least one atom may be the atom of the multiplet or a different atom comprised within the respective element - that is located at a distance equal to or less than a threshold "neighbouring distance" from at least another atom of another element comprising an atom of the multiplet - wherein the at least another atom may be an atom of the multiplet or a different atom comprised with the respective another element; and (ii) the distance between at least one pair of atoms comprised within different elements is equal to or less than a "contacting distance". For example, in the case of multiplets of atoms comprised within amino acid backbones or amino acid residues, the neighbouring distance may be 18 Å, while the contacting distance may be 4.5 Å.

Each one of the multiplets in the database comprises an identity of each element comprising each one of the atoms in the multiplet, and an associated geometrical property. In other words, the database comprises multiplets of atoms, each of which belongs to a different neighbouring element, wherein at least two of the atoms comprised within said neighbouring elements are within a contacting distance from each other, and the database also comprises the associated geometrical property of each multiplet. In the context of the present disclosure, the neighbouring distance is equal to or greater than the contacting distance.

Once the "matches" have been found, the CI method comprises determining the identity of the amino acid residue to be assigned to the reference position in the polypeptide backbone. Such identity is the identity of an element deemed to interact, i.e., an element that comprises an atom of a multiplet found in the database, said element being the element comprising the atom of the matching multiplet that corresponds, according to the ordered relationship, to the atom of the reference multiplet comprised in the amino acid backbone in the reference position or in the amino acid residue in the reference position. In other words, the atom corresponding to the atom in the reference position, according to the ordered relationship, may, for example, comprise that the match has been found for a geometrical property in a specific position in space and that such position in space determines which is the element whose identity should be assigned to the reference position in the polypeptide backbone. One or more identities may be selected or proposed for the identity assignment.

Once the identity or identities are determined, the CI method comprises assigning the identity of the amino acid residue to the reference position in the polypeptide backbone. Assigning may comprise generating a sequence or modelling a structure for the polypeptide backbone, which comprises, in the reference position, an amino acid residue with a side-chain according to the determined identity. The CI method may comprise assigning one or more identities which may have been determined for the reference position in the polypeptide backbone.

In examples, the multiplet being a triplet or a quartet allows determining matches considering sufficient or required information about the actual structure, e.g., three-dimensional structure, of interacting elements in a macromolecular structure. In a particular example, the multiplet being a triplet or a quartet allows determining matches on the basis of interaction data for at least 3 amino acid residues in a macromolecular structure, which provide valuable three-dimensional information that takes into account protein folding. In particular, triplets provide an adequate compromise between the information associated with a reference multiplet and the depth of variability of multiplets of atoms in a database. In other words, triplets incorporate context regarding the folding adopted by a polypeptide backbone and the relative positions of the atoms in space. Conversely, the variability of multiplets of atoms in a database generated from empirically-derived, high-resolution data of macromolecular structures is inversely correlated with the number of atoms in a multiplet. Thus, the higher the number of atoms per multiplet, the shallower the information of the database. In other words, the number of atoms in a reference multiplet is inversely proportional to the number of potential matching mutiplets that may be found in a database and which may be matched to a particular geometry. Thus, starting from a reference triplet allows exploring a high number of matching triplets, observed in empirical macromolecular structures, without losing 3-dimensional information. To further illustrate this, in a scenario where all elements of a multiplet are amino acid residues comprised within one or more polypeptides, two residues interacting with each other in isolation would undergo a relatively elevated entropic cost for forming the interaction, so that they would not gain the interaction energy necessary for estabilising the interaction. As such, in nature, it is common for proteins to have 3 residues in contact, one of which pays the entropic cost, while the other two provide the necessary energy gain. Thus, in the context of the present disclosure, the multiplet being a triplet or a quartet advantageously represents empirical interactions with greater accuracy.

The CI method may be used in cases where a part or all of the amino acid sequence of a polypeptide is not known or is to be designed from scratch. The CI method may also be used in cases where the amino acid sequence of a polypeptide is known but the identity of the amino acid residue in one or more reference positions is to be substituted by another identity.

In another aspect of the disclosure, there is disclosed a computer program comprising instructions which, when the program is executed by a computer, cause the computer to carry out the computer-implemented method of the disclosure.

In another aspect of the disclosure, there is disclosed a macromolecular structure comprising a polypeptide comprising an amino acid residue in a reference position, the amino acid residue having an identity assigned by the computer-implemented method according to the disclosure.

In a further aspect of the disclosure, there is disclosed a method to produce a macromolecular structure, the macromolecular structure comprising a polypeptide comprising an amino acid residue in a reference position, the amino acid residue having an identity assigned by the computer-implemented method according to the disclosure.

### FIGURES

Figure 1 shows a schematic view of an example of a macromolecular structure 101, the macromolecular structure comprising a polypeptide backbone 103 comprising a reference position 102, the polypeptide comprising an amino acid backbone 105 and an amino acid residue side-chain 104.
Figure 2 shows a polypeptide backbone 202 to which an identity of an amino acid residue is to be assigned.
Figure 3 shows a schematic representation of the implementation of some steps of the CI method of the disclosure.
Figure 4 shows a representation of matching multiplets.
Figure 5A shows an example of scoring of elements deemed to interact.
Figure 5B shows different triangles having an edge in common formed by atoms in positions 501 and 502.
Figure 5C shows an example of scoring of elements deemed to interact.
Figure 6 shows combinations of identities assigned to SH3 domain core residues through implementing the CI method of the present disclosure.

### DETAILED DESCRIPTION

### Definitions:

A "molecule", as used herein, refers to a group of two or more atoms that are linked together by covalent chemical bonds. Molecules may be homonuclear - the atoms comprised in a molecule may be the same, e.g., an oxygen molecule has two oxygen atoms -or may be heteronuclear - the atoms comprised in a molecule may be different, e.g., a water molecule has two hydrogen atoms and one oxygen atom. An "organic molecule", as used herein, refers to a molecule that comprises carbon atoms covalently bound to hydrogen atoms, C-H bonds, and may additionally comprise further chemical elements, including, but not limited to, oxygen, nitrogen, sulfur, and phosphorous. An "inorganic molecule", as used herein, refers to a molecule that does not comprise carbon atoms covalently bound to hydrogen atoms, and which comprises two or more chemical elements.

"Macromolecular structure", as used herein, refers to one or more molecules whose atoms are organised in a three-dimensional arrangement. The macromolecular structure may consist of a polypeptide, a polypeptide backbone, or a protein, or it may comprise a macromolecular complex comprising two or more interacting molecules, e.g., two interacting proteins, an antibody-antigen complex or part of an antibody-antigen complex comprising the antibody-antigen interface, one or more polypeptides interacting with non-protein molecules, including, but not limited to, nucleic acids, lipids, carbohydrates, water molecules or other inorganic molecules, small molecule drugs, or combinations thereof.

An "element", as used herein, refers to a sub-component of a macromolecular structure and may comprise a small molecule, an inorganic molecule, or part of a biological macromolecule, such as a part of a lipid molecule, part of a carbohydrate molecule, part of a nucleic acid molecule, or part of a polypeptide, in particular, a subunit of a biological polymer, such as a nucleotide, a monosaccharide, an amino acid residue, or an amino acid residue backbone.

An "amino acid", as used herein, refers to a nitrogen-containing organic compound that comprises both an amino functional group and a carboxylic acid functional group. An amino acid may be a proteinogenic amino acid, or it may be a non-proteinogenic amino acid, such as a naturally occurring non-proteinogenic amino acid or a synthetic non-proteinogenic amino acid. A proteinogenic amino acid may be any of one the 22 known proteinogenic amino acids and may be referred to by its abbreviated three-letter name or by its abbreviated one-letter name, according to standard nomenclature. Thus, a proteinogenic amino acid may be selected from a list consisting of: Alanine (Ala or A), Cysteine (Cys or C), Aspartic acid (Asp or D), Glutamic acid (Glu or E), Phenylalanine (Phe or F), Glycine (Gly or G), Histidine (His or H), Isoleucine (lle or I), Lysine (Lys or K), Leucine (Leu or L), Methionine (Met or M), Asparagine (Asn or N), Pyrrolysine (Pyl or O), Proline (Pro or P), Glutamine (Gln or Q), Arginine (Arg or R), Serine (Ser or S), Threonine (Thr or T), Selenocysteine (Sec or U), Valine (Val or V), Tryptophan (Trp or W), and Tyrosine (Tyr or Y). An "amino acid residue", as used herein, refers to an amino acid moiety that is incorporated in an oligopeptide, a polypeptide or a protein, and is bonded to other amino acid residues by peptide bonds. In the context of the present disclosure, an amino acid comprised in the macromolecular structure may be an unmodified amino acid, or it may be a modified amino acid, such as, for example, an amino acid carrying a post-translational modification, including, but not limited to, phosphorylation, glycosylation, acylation, methylation, sulfation, lipoylation, palmitoylation, myristoylation, alkylation, amidation, glycation, sumoylation, and ubiquitination.

An "amino acid backbone" refers to the core structure that is common to all amino acids and comprises an amino group (NH2), a central (alpha) carbon, a carboxyl group (COOH), and a hydrogen atom. The skilled person understands that the "alpha carbon", "α-carbon", "carbon-α" or "Cα" of an amino acid residue or of an amino acid backbone, refers to the carbon atom in the central position of the backbone common to all amino acids, also known as the α-position, or alternatively the 2-positon, which is bonded to an amino group (NH2), a carboxyl group (COOH), a hydrogen atom and a "side-chain". An amino acid "sidechain" refers to an atom or group of atoms bonded to the alpha carbon, and which is different for every amino acid. The skilled person further understands that the "beta carbon", "β-carbon", "carbon-β" or "Cβ" of an amino acid residue, refers to the first carbon atom of an amino acid side-chain, if such carbon atom is present. If present, the beta carbon is bound to the alpha carbon.

A "polypeptide backbone", as used herein, refers to a macromolecule comprising a chain of amino acids backbones linked together by peptide bonds. The polypeptide backbone may be modified, for example at the N-terminus and/or at the C-terminus, through modifications that the skilled person is familiar with, or it may be conjugated with further functional moieties, including, but not limited to, a fluorophore, a linker, a signal peptide, or a small molecule. In a polypeptide backbone, amino acid side-chains may be attached to one or more of the amino acid backbones. A "polypeptide", as used herein, refers to a polymeric biological molecule comprising a chain of amino acid residues, also known as amino acid monomers, linked together by peptide bonds. Chains of fewer than twenty amino acids are called oligopeptides, and include dipeptides, tripeptides, and tetrapeptides. The skilled person understands that a peptide bond, also known as an amide bond, is a covalent chemical bond that links together the carboxyl group of one amino acid with the amino group of an adjacent amino acid, through a dehydration reaction. Thus, an oligopeptide, a polypeptide or a polypeptide backbone, comprises a free amino group, known as the N-terminus, and a free carboxyl group, known as the C-terminus.

The positions of amino acids residues or amino acid backbones, comprised within an oligopeptide, a polypeptide or a polypeptide backbone, are numbered sequentially starting from the N-terminus. Thus, the amino acid residue or amino acid backbone comprising the free amino group represents position number 1 of the respective oligopeptide, polypeptide or polypeptide backbone. Thus, as used herein, a "position" of an amino acid backbone or of an amino acid residue comprised within a polypeptide backbone, an oligopeptide or a polypeptide, refers to the position of said amino acid backbone or of said amino acid residue within the respective polypeptide backbone, oligopeptide or polypeptide.

A "reference position" as used herein refers to a position within a polypeptide backbone where the identity of the amino acid residue occupying said position is to be assigned. The skilled person understands that the identity of an amino acid is determined by its side-chain.

A "protein", as used herein, refers to a biological macromolecule that comprises one or more polypeptides.

A "starting" or "original" polypeptide or polypeptide backbone, as used herein, refers to a polypeptide or polypeptide backbone to which the identity of the amino acid residue(s) occupying one or more reference positions is to be assigned or substituted in the present disclosure.

A "final polypeptide" or "final polypeptide backbone", as used herein, refers to a polypeptide or a polypeptide backbone wherein the identity of an amino acid residue in at least one position is different to the identity of the amino acid residue or amino acid backbone found in the respective position in the "starting" or "original" polypeptide or polypeptide backbone. The final polypeptide or final polypeptide backbone may also be referred to as a polypeptide or final polypeptide "variant".

A "substitution" or "modification", as used herein, refers to assigning a different identity to an amino acid residue in a reference position of a polypeptide or a polypeptide backbone, wherein the identity of an amino acid residue is defined by its side-chain. A "conservative amino acid substitution", also known as also called a "conservative mutation", a "conservative replacement" or a "homologous replacement", as used herein, refers to changing the identity of the original amino acid residue occupying a reference position to a different identity, wherein the different identity is that of a different amino acid with similar biochemical properties (e.g. charge, hydrophobicity and size) to the original amino acid residue.

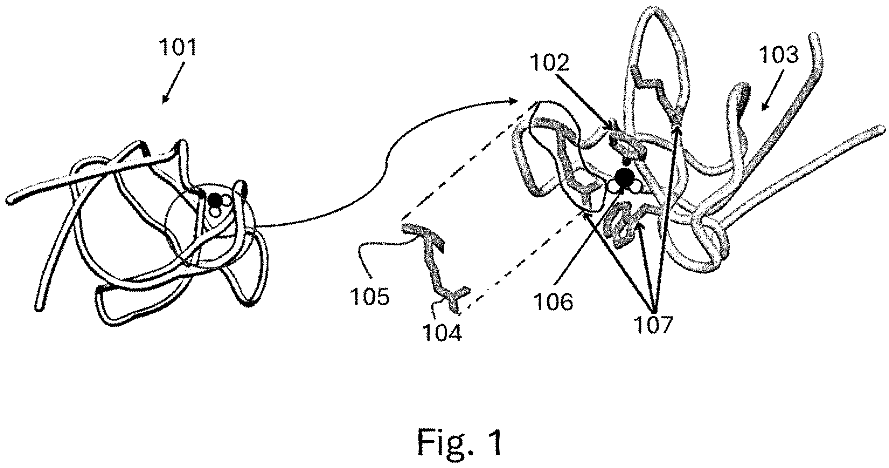

Figure 1 shows a schematic view of an example of a macromolecular structure 101, the macromolecular structure comprising a polypeptide backbone 103 comprising a reference position 102, the polypeptide comprising an amino acid backbone 105 and an amino acid residue side-chain 104.

The macromolecular structure 101 comprises a plurality of elements , the elements comprise: an amino acid residue in the reference position 102; and one or more neighbouring elements 107. In the example of figure 1 the neighbouring elements comprise a small molecule being a water molecule 106. Other neighbouring elements 107 are shown in figure 1 which comprise further amino acid residues.As explained herein, the neighbouring elements comprise at least one atom within an neighbouring distance from at least one atom of the amino acid residue in the reference position 102.

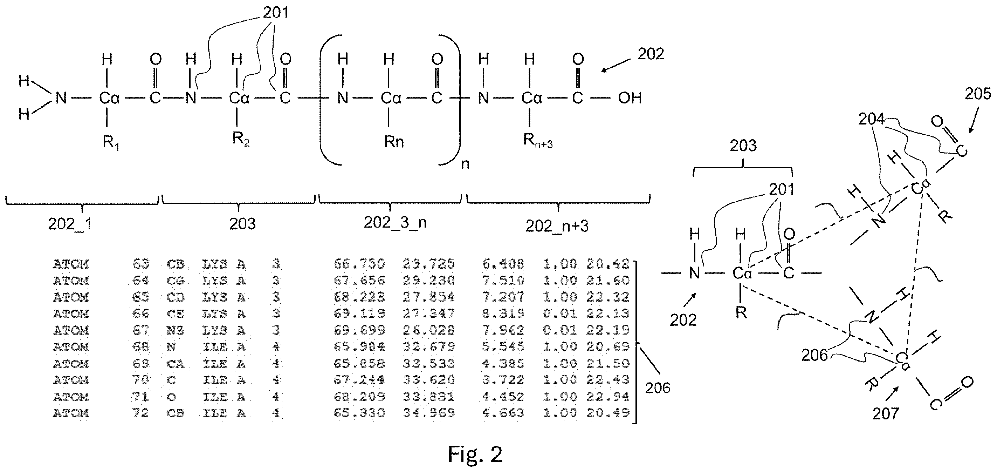

Figure 2 shows a polypeptide backbone 202 to which an identity of an amino acid residue is to be assigned. The polypeptide backbone comprises different amino acid backbones or amino acid resides in different positions, the position 1 or N terminus is referenced as 202_1, the position n+3 or C terminus is referenced as 202_n+3, the reference position is referenced as 203 and the rest of positions between 203 and 202_n+3, i.e., positions 3 to n+2, are referenced as 202_3_n. In figure 2, the R groups represent the amino acid residue side-chains. In the case of a polypeptide backbone the side-chains are absent. Figure 2 shows atoms 201 comprised in the polypeptide backbone 202 within the reference position 203. Figure 2 further shows a schematic view of the polypeptide backbone 202 in figurative contact by a dashed line with neighbouring elements 205 and 207 which are represented as different neighbouring amino acid residues 205 and 207. Figure 2 shows the atoms 204 within the neighbouring elements 205 and the atoms 206 within the neighbouring elements 207. The CI method comprises, with reference to figure 2, obtaining the reference position 203 and obtaining spatial coordinates 206 of at least one atom 201 comprised in the polypeptide backbone 202 within the reference position 203.

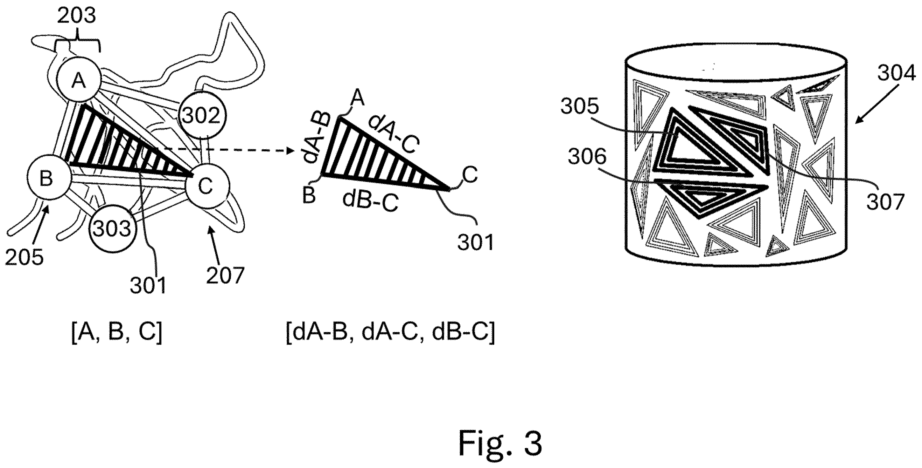

Figure 3 shows a schematic representation of implementation of some steps of the CI method of the disclosure. With reference to figures 2 and 3, the reference position 203 may be obtained or received. In examples, the reference position 203is read or obtained from a file-Atomic coordinates may be obtained from the file so that the CI method may proceed determining the reference multiplet of atoms, which in case of figure 2 is a triplet of atoms [201, 204, 206] and which in case of figure 3 is a triplet of atoms [A, B, C]. In figure 3, the reference triplet consists of one atom A comprised in the amino acid backbone in the reference position 203 or the amino acid residue in the reference position 203, one atom B of a neighbouring amino acid residue 205 and the atom C of a neighbouring amino acid residue 207. The reference triplet [A, B, C] is associated to a reference geometrical property, which in the case of figure 3 the reference geometrical property is a triplet of distances [dA-B, dA-C, dB-C] of the edges of a triangle 301 defined by the atoms A, B, C representing vertices of the triangle 301, and which triangle 301 is represented by a pattern with lines. The distance between the atoms of the reference triplet comprised within each pair of two or more neighbouring elements, e.g., the distances dA-B, dA-C, dB-C, are, each, equal to or less than a neighbouring distance. If the atom 201 in figure 2 or atom A in figure 3 is one of the Cα atom 201 or the carbon beta atom then the neighbouring element 205 comprises at least one of its Cα atom 204 or the carbon beta atom within a neighbouring distance, e.g., 18 A from the Cα atom 201 or the beta carbon atom of the polypeptide backbone 202 respectively. Figure 3 shows the atom 302 and the atom 303 which belong to different neighbouring elements from the elements 205 and 207. The CI method may also determine a second reference triplet comprising atoms [A, 209, C] and a third reference triplet comprising atoms [A, B, 210]. As seen, in the example of figure 2, the at least one matching multiplet of atoms comprises atoms of the same type as the type of the atoms of the reference multiplet, in the case of figure 2 being the C*α* atoms, but may also be C*β*.

The CI method further comprises, iteratively, per each one of the determined reference multiplets, or triplets, finding, in a database , at least one matching multiplet of atoms. The figure 3 shows the database 304 comprising a plurality of triplets of atoms which are represented as the vertices of the triangles they form. The triangles 305, 306 and 307 are found to be a plurality of 3 matching triangles associated to a plurality of matching triplets. As seen, the CI method may comprise finding a plurality of matching multiplets. The atoms in each matching multiplet are ordered according to an ordered relationship between atoms, where the ordering allows keeping a record of a position in at least a 2-dimensional plane of each atom relative to the rest of the atoms in the multiplet. The position in at least a 2-dimensional plane may indicate an orientation in space of the atoms in the polygon, e.g., triangle, defined by each multiplet, e.g., triplet. When finding the match, the ordering of the atoms may be varied by varying the atom that occupies a first position of the ordered relationship, changing thereby the orientation or rotation of the polygon,. Varying the ordering allows, therefore, and with reference to the illustrative example of figure 3 showing triangles 305, 306 and 307, finding matches of triplets, where the triplets match regardless of the original orientation of the triangles they form, since the ordering or orientation of triangles may be varied for finding the match. In other words, the triangles may be reoriented or rotated for finding a match in the database.

In the case of figure 3, the database 304 comprises triplets of atoms within elements deemed to interact in empirical macromolecular structures and within elements deemed to interact in virtually generated models of macromolecular structures, and, for each one of the triangles: the database 304 comprises an identity of each element comprising each one of the atoms, and an associated geometrical property, which in the case of the triangles 305, 306, 307 may be the distance of the edges. Other geometrical properties may be chosen such as the angles formed by the edges. Whichever the reference geometrical property, the associated geometrical property comprises the same type of units (i.e., angles or distances). The associated geometrical properties of the matching triplets substantially equal the reference geometrical property [dA-B, dA-C, dB-C] of the reference triplet [A, B, C], which is represented as matching triangles 305, 306 and 307 of the database 304 "fitting in" or presenting substantially the same geometrical shape as the reference triangle 301, regardless their orientation in the database 304.

In examples, the associated geometrical property of the at least one matching multiplet of atoms comprises one or more distances between at least one pair of atoms comprised within a respective pair of elements, which comprise the atoms of the at least one matching multiplet being equal to or less than the contacting distance. In other words, the matching may, in some examples, be allowed only if a filter based on the number of contacts is passed, the filter comprising selecting as matching multiplets, or triplets in figure 3, only the multiplets forming polygons or forms with a certain number of "internal" contacts or interactions; in some examples having "internal" contacts may comprise that at least one atom in one polygon or form -or triangle-is distanced from a different atom in a different polygon or form -or triangle- by a contacting distance, e.g., 4.5 Å.

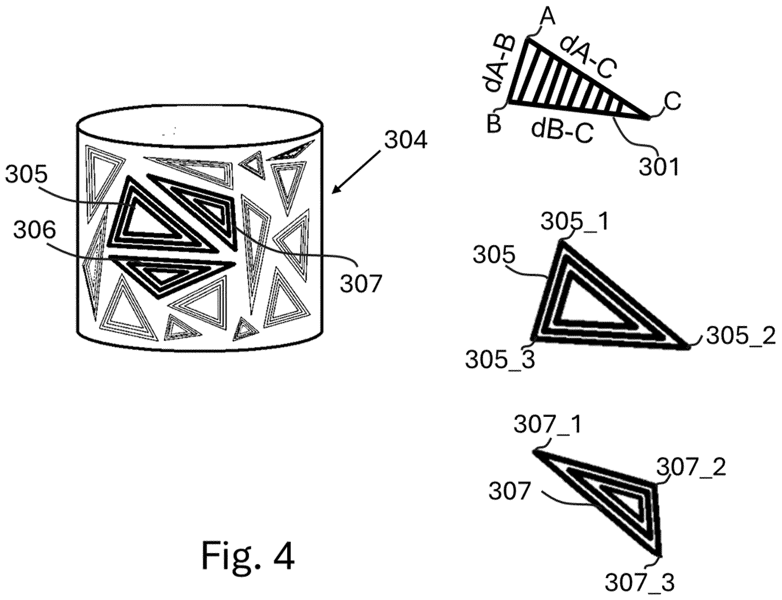

The CI method further comprises determining the identity of the amino acid residue as the identity of an element deemed to interact comprising the atom of the at least one matching multiplet which corresponds, according to the ordered relationship, to the atom of the reference multiplet comprised in the amino acid backbone in the reference position or in the amino acid residue in the reference position. Figure 4 shows a representation of matching multiplets. With reference to figure 4, the reference position to which an identity is to be assigned is the position in which atom A of amino acid residue 301 is comprised. The ordered relationship of the matching triplets [305_1, 305_2, 305_3] and [307_1, 307_2, 307_3] is an order in which the geometrical properties are substantially equal. In examples, only the matching triplets may be ordered. In these examples the matching triplet associated with triangle 305 is ordered as [305_1, 305_3, 305_2] to match -in orientation so that the edges can "match" or "fit"- the reference triplet [A, B, C] and the matching triplet associated with triangle 307 is ordered as [307_3, 307_2, 307_1]. In examples both the reference triplet and the matching triplets may be ordered. In these examples, the reference triplet may, for example, be ordered such that the geometrical property [dA-B, dA-C, dB-C] allocates the first position to the smallest distance, resulting in an ordering such as [dAB, dB-C, dA-C], which results in the triplet being ordered as [A, B, C]; the triplet associated with triangle 305 results ordered as [305_1, 305_3, 305_2] to match the reference triplet [A, B, C] and the triplet associated with triangle 307 results ordered as [307_3, 307_2, 307_1]. In the case of the triplets of figure 4, the atoms which correspond, according to the ordered relationship, to the atom A of the reference multiplet comprised in the amino acid backbone in the reference position or in the amino acid residue in the reference position are 305_1 and 307_3. The element deemed to interact comprising the atom 305_1 may be, for example, Alanine, and Alanine would therefore be identified as the identity of the amino acid residue or amino acid backbone in the polypeptide backbone within the reference position. The element deemed to interact comprising the atom 307_3, may be, for example, Valine, and Valine would therefore be identified as the identity of the amino acid residue or amino acid backbone in the polypeptide backbone within the reference position. In this case there exist two possible candidates for the identity: Ala, and Val, in which case, the CI method may provide two possible results or may implement a scoring between the two, as explained herein after.

The CI method finally comprises assigning the identity or identities of the amino acid residue to the reference position in the polypeptide backbone, providing one or more possible polypeptides or final polypeptides.

The resulting or final polypeptides may be specifically designed for improving a stability of an original polypeptide or for providing other improved properties, e.g., affinity, specificity, aggregation, half-life, controlling subcellular localisation, affecting function through post-translational modifications.

In examples, the CI method comprises blocking or predefining or setting an identity for one or more of the elements deemed to interact, so that only multiplets comprising atoms that belong to elements with one or more predefined identities selected from a list of predefined identities may be selected as matching multiplets. The atom of the element with the predefined identity is comprised in the matching multiplet of atoms in a predefined position within the ordered relationship. The predefined identities may be referred to as "allowed identities". Predefining or blocking or setting the identities of elements in this manner allows designing improved polypeptides in terms of, for example, glycosylation or phosphorylation, since different post-translational modifications are attached to specific amino acid residue side-chains. The identity of the one or more elements deemed to interact may be predefined based on the identity of the neighbouring elements that comprise the atoms of the reference multiplet.

In some examples, the identity of the assigned amino acid residue has different biochemical properties from the biochemical properties of the amino acid residue in the reference position, wherein the different biochemical properties may include one or more of charge, hydrophobicity and size, such that substituting the amino acid residue in the reference position with the assigned amino acid residue does not result in a conservative substitution. These examples advantageously allow providing a (highly) different polypeptide than an original polypeptide in the case where an identity of an amino acid residue in the reference position is to be substituted and these examples advantageously allow providing a polypeptide with specific properties in the case where the original polypeptide has no amino acid residue in the reference position.

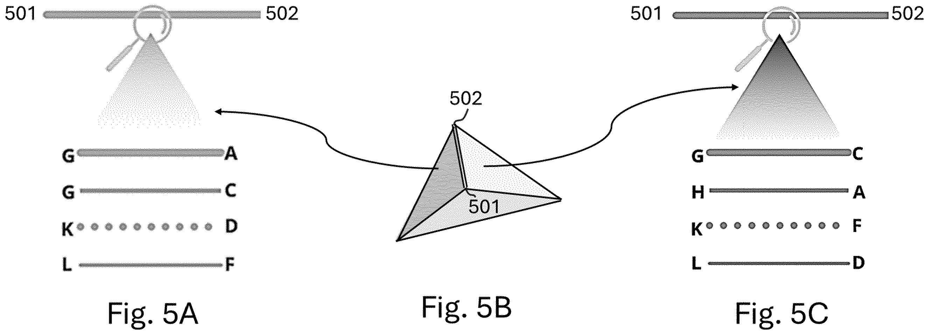

In some examples, determining the identity of the amino acid residue comprises a scoring of elements deemed to interact, the elements deemed to interact comprising the atom of the at least one matching multiplet. In other words, in examples there may be several identities which are determined as possible identities to be assigned. A solution to choose one or more of them may be made by applying a scoring. Figures 5A, 5B and 5C show an example of scoring of elements deemed to interact. Figure 5B shows different triangles having an edge in common formed by atoms in positions 501 and 502. Figures 5A and 5B show the different triangles with a common edge. In figure 5A, one of the triangles of figure 5B is shown. In figure 5A different matching triplets -found in the database-, comprise an atom which coincides with/corresponds to the atom comprised in the amino acid backbone in the reference position or in the amino acid residue in the reference position, indicated as the position 501. In figure 5A, the atoms which coincide with the atom comprised in the amino acid backbone in the reference position or in the amino acid residue in the reference position 501 are comprised within the neighboring elements Glycine -G, Glycine- G, Lysine -K and Leucine - L. In figure 5A, the matching triplets comprise an atom which coincides with the atom indicated in position 502. The atoms are comprised within Alanine - A, Cysteine - C, Aspartic acid -D and Phenylalanine - F. In figure 5C, one of the triangles of figure 5B is shown. Using the same atoms indicated in positions 501 and 502 in figures 5A and 5C, in figure 5C, other different matching triplets -different from triplets in figure 5A- comprise atoms which coincide with the atom comprised in the amino acid backbone in the reference position or in the amino acid residue in the reference position, indicated as the position 501. These atoms are comprised within the neighbouring elements Glycine -G, Histidine - H, Lysine -K and Leucine - L. In figure 5C, the other different matching triplets comprise atoms which coincide with the atom indicated in position 502. These atoms are comprised within Cysteine - C, Alanine - A, Phenylalanine - F and Aspartic acid -D. The scoring may comprise determining the amino acid residue frequencies in the results. For example, for the residue position 501, and considering all the elements of figures 5A and 5C, G appears 3 times, K appears twice, L appears twice and H appears once. Then for example, G might be chosen as the identity to be assigned since G is the more frequent in the matches. In other example, the scoring may comprise adding the frequencies of combinations of amino acid residues, which may result in considering that, for the atom in position 501 considering both figures 5A and 5C, the combination G-A appears once, the combination G-C appears twice, the combination K-D appears once, and the rest of combinations appear once. Then the chosen identity to assign may be G and the identity of the amino acid residue comprising atom in position 502 may be C, since G-C is the more frequent combination of elements in positions 501 and 502.

Other scoring may be possible, such as a statistical scoring function, a neural network scoring function or scoring based on random forest any other.

In examples the CI method comprises predicting a three-dimensional, 3D, structure of a polypeptide comprising the polypeptide backbone with the assigned identity of the amino acid residue in the reference position. The CI method may predict the three-dimensional structure of a model of the polypeptide which has been designed by assigning an identity of an amino acid residue to the reference position of the polypeptide backbone. The CI method may comprise steps for predicting the 3D structure or the CI method or for sending the model of the polypeptide to a specific computer program to have the 3D structure predicted. Some computer programs which may predict the 3D structure may comprise AlphaFold, Esm, RoseTTAFold, Chai-1 . In these examples, there is presented a CI method to predict a three-dimensional structure of a -model of-a polypeptide.

In examples the CI method comprises estimating the stability of the polypeptide. The CI method may estimate the stability of a model of the polypeptide which has been designed by assigning an identity of an amino acid residue to the reference position of the polypeptide backbone. The CI method may comprise steps for predicting the stability or the CI method or for sending the model of the polypeptide to a specific computer program to have the stability estimated. In these examples, there is presented a CI method to estimate the stability of a -model of- a polypeptide. Some computer programs to estimate stability may comprise FoldX, Rosetta, DDgun, GROMACS, I-TASSER.

In some examples, the present disclosure presents one or more computer programs comprising instructions which, when the program(s) is (are) executed by a computer, cause the computer to carry out the CI method of any one of the examples in the present disclosure.

In some examples the present disclosure presents a macromolecular structure comprising a polypeptide comprising an amino acid residue in a reference position, the amino acid residue having an identity assigned by the CI method of any one of the examples in the present disclosure.

In some examples, the present disclosure presents a method to produce a macromolecular structure, the macromolecular structure comprising a polypeptide comprising an amino acid residue in a reference position, the amino acid residue having an identity assigned by the CI method of any one of the examples in the present disclosure.

### The database

The database from which matching multiplets are to be retrieved, may be accessed locally or by means or any intermediate switching point such as a network device, such as a router, a gateway, a firewall or an access point. The database may be stored in a non-transitory storage means. The database may be created or generated. The CI method may comprise creating or generating the database. The database may be generated by implementing, in an example, the following steps.

A representative set of -digital- models of crystal structures may be provided. For example, a set of 5095 crystal structures, and for example high resolution crystal structures, for example representing the structures in units of 1,5 Å or less. Entries with more than 95% of similarity may be reduced to one representative. Every model in the set may be represented in multiplets, e.g., triplets, of interacting elements, e.g., residues, for which geometrical properties may be registered into one or more entries of a database. A first set of multiplets, e.g., triplets, may be determined when at least one atom of each amino acid of the multiplets, e.g., triplets, may find a backbone from the other (two) elements, e.g., residues, within a sphere of a neighbouring distance. The neighbouring distance between two interacting residues may be 18 Å, the largest Cα-Cα distance observed in the database,. The first set of multiplets, e.g., triplets, may later be pruned to those having at least one couple of atoms within interacting or contacting elements, e.g., at less than 4.5 Å. When the multiplets, e.g., triplets, of elements, e.g., residues, are identified within a crystal structure, the set of multiplets, e.g. triplets, may be registered together with the number of contacts between the elements comprised in each multiplet, e.g., residues, and the identities of such elements. The distances may be distances between pairs of α and β carbon of the three elements, e.g., residues. Three levels of contact may be assigned to stratify the population of multiplets or triplets or triangles composing the database: one contact if at least one atom from one elements, e.g., residue side chain, stays within 4.5 Å of any atom of any of the other residues. In the case of triplets, triangles with less than 1 contact may not be included in the database. Two and three contacts may follow the same rule, requiring respectively two and three different side chains to contact at least another one within the triplet.

In examples, 60,563,237 triplets are determined, and their geometrical properties are determined or calculated.

Any of the CI method of the disclosure may be implemented by one or more processing units. The processing units of the present disclosure may comprise or may be implemented by electronic means, computing means or a combination of them, that is, electronic or computing means may be used interchangeably so that a part of the described means may be electronic means and the other part may be computing means, or all described means may be electronic means or all described means may be computing means. Examples of a processing unit comprising electronic means may comprise a programmable electronic device such as a Complex Programmable Logic Device, CPLD, a Field Programmable Gate Array, FPGA or an Application-Specific Integrated Circuit, ASIC.

Examples of a processing unit comprising computing means may comprise a computer system, which may comprise a memory and a processor, the memory being adapted to store a set of computer program instructions, and the processor being adapted to execute the computer program instructions stored in the memory or in a non-transitory readable storage medium. The memory may be comprised in the processing unit, for example an EEPROM, or may be external, for example, data storage means such as magnetic disks, e.g., hard disks, optical disks, e.g., DVD or CD, memory cards, flash memory, e.g., pen drives, or solid-state drives, SSD based on RAM, based on flash, etc. A set of computer program instructions may be executable by the processor, such as a computer program, may be stored in a physical storage means, or may be carried by a carrier wave, or by a carrier medium. The processing unit may be any entity or device capable of carrying the program, such as electrical or optical, which can be transmitted via electrical or optical cable or by radio or other means.

The computer program may be in the form of source code, object code, a code intermediate source and object code such as in partially compiled form, or in any other form suitable for use in the implementation of the method. The carrier may be any entity or device capable of carrying the computer program.

The computer program may be embodied on a storage medium (for example, a CD-ROM, a DVD, a USB drive, a computer memory or a read-only memory) or carried on a carrier signal (for example, on an electrical or optical carrier signal).

### Examples

In an example, a protein design method comprises the computer-implemented method, CI method, to assign identities to amino acid residues occupying reference positions in a polypeptide backbone according to the present disclosure, in order to determine candidate variants of said polypeptide. The protein design method comprises a redesign of a hydrophobic core of a SRC Homology 3 domain, SH3 domain, of the spectrin cytoskeletal protein from *Gallus gallus,* a domain which consists of 62 residues. The hydrophobic core of said spectrin SH3 domain includes nine hydrophobic residues, identified as residues with a solvent accessibility of less than 10%. These residues are Val 9, Ala 11, Val 23, Met 25, Leu 31, Val 44, Val 46, Val 53 and Val 58 (PDB ID: 1shg). The amino acids in all nine reference positions are virtually mutated to Alanine prior to implementing the CI method, to eliminate any biases imposed by the rotamer placement constraints of the wildtype side-chains. Thus, the starting polypeptide comprises the amino acid sequence of the *Gallus gallus* spectrin SH3 domain wherein Alanine is present at positions 9, 11, 23, 25, 31, 44, 46, 53, 58. The macromolecular structure comprises the starting polypeptide wherein the atoms of the elements, i.e., amino acid residues, are organised in three-dimensional space according to the structure of the wildtype polypeptide as recorded in PDB ID: 1shg. The CI method obtains the reference positions 9, 11, 23, 25, 31, 44, 46, 53, 58. The CI method obtains the spatial coordinates of atoms comprised within each of the 62 amino acid residues of the spectrin SH3 domain from the PDB data file. The CI method may implement the steps of assigning an identity iteratively, each reference position per iteration. The CI method may implement the steps of assigning an identity in parallel, or at the same time.

The CI method is implemented to assign identities to one or more of the nine core amino acid residues occupying the reference positions in the SH3 hydrophobic domain, wherein at least one of the identities is different from the identity of the amino acid residue occupying the respective reference position in the original SH3 domain polypeptide.

For this, the CI method determines reference triplets of atoms, wherein at least one of the atoms of the triplet is comprised within an amino acid residue occupying a reference position. The remaining atoms in the triplet are comprised within neighbouring amino acid residues, which may occupy one or two other reference positions or may occupy a non-reference position. The reference triplets are triplets of carbon-α, Cα, atoms or triplets of carbon-β, Cβ, atoms. Thus, for every combination of three amino acid residues, two reference triplets of atoms are determined, each of which are associated with a respective reference geometrical property. Thus, the reference geometrical properties comprise triplets of Cα-Cα distances and triplets of Cβ-Cβ distances, respectively. In other words, for every combination of three amino acid residues, such reference geometrical properties describe the features associated with triangles formed by Cα-Cα-Cα atoms and by Cβ-Cβ-Cβ atoms, respectively.

For every refence triplet of atoms, the CI method finds at least one matching multiplet, e.g., a matching triplet, of atoms in a database. Finding a matching multiplet, e.g., a triplet, of atoms comprises finding triplets of Cα-Cα-Cα atoms or of Cβ-Cβ-Cβ atoms, as applicable, in the database, which form substantially identical triangles to the triangles formed by the atoms of the reference multiplet. This may be implemented by superimposing triangles formed by the union of reference triplet of Cα-Cα-Cα atoms or of Cβ-Cβ-Cβ atoms allowing a tolerance, for example, a tolerance ranging from about 0.1 Å to about 0.5 Å, for example a tolerance of about 0.3 Å, to account for potential small shifts in backbone structure. Finding a matching triplet comprises computing an ordered relationship for the atoms of the matching triplet such that each atom of the matching multiplet correlates with/corresponds to one atom of the reference multiplet in a specific orientation in a 2D plane or in space. In other words, matching two triangles based on their geometrical features entails also establishing a correlation between their respective vertices in a specific orientation in a 2D plane or in space. Triplets in the database derived from macromolecular structures with a sequence similarity greater than a threshold, for example, a 30% sequence similarity, compared to the original SH3 domain polypeptide sequence may be excluded, to ensure that the identities assigned to the amino acid residues in the reference positions diverge from those of the amino acid residues present in the original wildtype SH3 polypeptide.

A pre-existing database is used, the database having been generated by *in silico* digesting a representative set of 5,095 high-resolution, <1.5 Å, crystal structures from the Protein Data Bank. The database contains triplets of atoms comprised within amino acid residues that interact in empirically-observed macromolecular structures. The database may be generated by a method as explained in the present disclosure.

The CI method determines at least one possible identity for an amino acid residue in each refence position of the SH3 hydrophobic core domain based on the identity of the amino acid residue comprising the atom of the matching triplet that occupies the respective position in the ordered relation. Where more than one matching triplet is found, several possible identities may be determined. In such a case, a scoring of the possible identities may be implemented, for example, based on the frequency of each possible identity as indicated by the frequency of matching atoms comprised within amino acid residues of each identity in the corresponding position of the ordered relationship. Determining a reference triplet of atoms, wherein the three atoms are comprised within amino acid residues at three reference positions, and assigning identities to all three residues enables the simultaneous placement of multiple side chains that are compatible with the backbone conformation of the starting SH3 polypeptide. As the hydrophobic core of spectrin SH3 domain is redesigned or varied, the identities of the amino acid residues comprising atoms of the matching multiplets, e.g., triplets, may be pre-determined or restricted. For example, while finding matching triplets of atoms in the database, triplets of atoms of certain characteristics may be excluded, e.g., by excluding triplets wherein the atom or atoms in predefined positions in the ordered relationship are comprised within charged residues (Asp, Glu, Lys, Arg), large polar residues (Asn, Gln), or Glycine. In other words, the identities of charged residues (Asp, Glu, Lys, Arg), large polar residues (Asn, Gln), or Glycine may be predefined as unallowed identities. Alternatively, or additionally, allowed or required identities may also be defined. For example, Histidine (His) may be included as such an allowed or required identity, as its side-chain can form favourable stacking interactions with aromatic residue side-chains. A further limiting condition may be defined that the CI method retrieves only triplets of atoms comprised within amino acid residues that have at least one, for example two, side-chain contacts, optionally in combination with allowed and/or unallowed identities of the amino acid residues to be assigned.

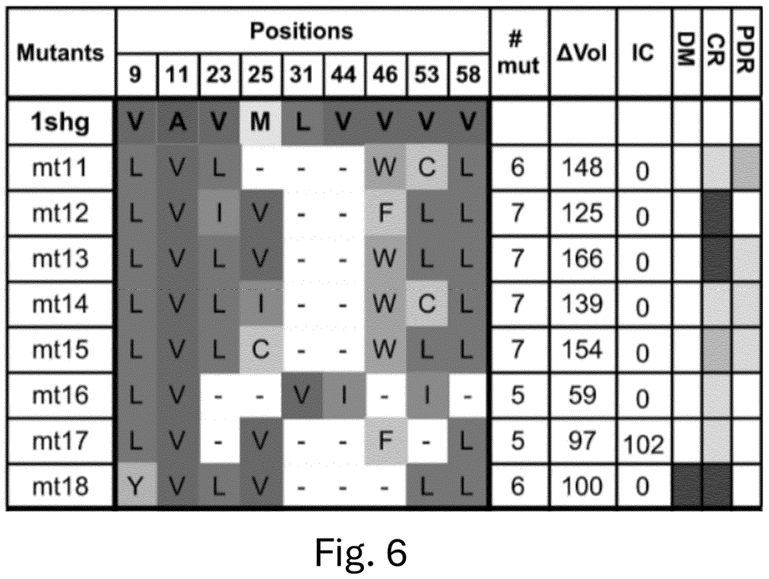

The CI method assigns at least one identity to at least one amino acid residue in a reference position of the SH3 domain polypeptide. The final polypeptide comprises amino acid residues of the same identities as those of the original polypeptide in non-reference positions and at least one amino acid residue in a reference position whose identity is different to the identity of the amino acid residue occupying the respective reference position in the original polypeptide. The identities assigned by implementing the CI method of the present disclosure are further used to provide 8 final polypeptides, also known as variants of the SH3 starting polypeptide, that comprise different combinations of amino acid residues in the nine reference positions of the SH3 core hydrophobic domain. These are given the names of mt11 to mt18. As shown in Figure 6, the 8 final SH3 domain polypeptide variants differ from the original polypeptide in at least 5 of the 9 reference positions are selected for further analysis. Figure 6 shows the identities of the amino acid residues present in the wildtype SH3 domain, 1shg, in each of the 9 reference positions of the hydrophobic core, namely: positions 9, 11, 23, 25, 31, 44, 46, 53 and 58. Figure 6 further shows combinations of identities assigned to each position through implementing the CI method of the present disclosure. The "-" symbol indicates that the identity of the amino acid residue occupying the respective position has not changed with respect to the wildtype, 1shg, sequence. From left to right, the columns of the table shown in Figure 6 indicate: (a) name of the final polypeptide variant (except for 1shg, the name of the original polypeptide); (b) identities of the assigned residues (or, in the case of 1shg, of the residues found in the original polypeptide); (c) number of assigned residues in each final polypeptide variant whose identities differ compared to the wildtype sequence for the mutant (# mut); (d) predicted variation in volume (Å³) for the hydrophobic core (ΔVol); (e) total cavity volume generated in Å (IC); (f) whether the mutant has total energy sensibly worse than its reference structure (DM); (g) presence of residues with van der Waals clashes (CR); (h) presence of residues with polar atoms desolvated that did not make an Hbond (PDR). Changes in the thermodynamic stability, i.e. ΔΔG, of final polypeptides compared to the original wildtype, 1shg, polypeptide are indicated with shades of grey. Light grey indicates at least one core residue with a ΔΔG with respect to the structure of the original SH3 domain polypeptide between 0.5 and 0.8 kcal/mol. Dark grey shows more than one residue between 0.5 and 0.8 kcal/mol, or at least one higher than 0.8 kcal/mol. Black boxes indicate more than one core residue above 0.8 kcal/mol.

The possible final polypeptide variants comprising different combinations of core residue identities may be scored, for example, based on similarity to or divergence from the original polypeptide, predicted stability or folding. Scores for the top 500,000 variants may be stored in relative output files. The top 10,000 ranking sequences for final polypeptides may be modelled over the structure of the original polypeptide, PDB ID: 1shg, using an algorithm, for example FoldX BuildModel, also allowing the replacement of a maximum of 2 clashing residue substitutions by utilizing the FoldX PSSM command to explore amino acid point mutations that may improve the stability of the final polypeptides.

To ensure the suggested combinations of amino acid which were not previously observed in nature: a sequence search within the SH3 domain family may be performed and each of the 8 final polypeptide variants/mutants may be aligned with the "full family" alignment for SH3_1 Pfam family ID:PF00018. The results include that out of the 8 identified final polypeptide variants, none matches previously reported SH3 domain variants. Instead, of the 8 final polypeptide variants, those with sequences most similar to any of the 92,969 known SH3 domain sequences shared only 6 out of 9 amino acid residues, e.g. mt14.

The eight variants or candidates may be chosen for experimental validation based on the following criteria: i) Candidates free of structural issues within the backbone of input; ii) Candidates potentially affected by minor structural issues ; iii) Candidates with strong structural issues (He qui. Structural issues may be considered, including: Van der Waals' clashes, cavities, hydrophobic desolvation and unsatisfied polar groups.

The 8 final polypeptide variants, alternatively known as SH3 domain mutants, and the original wildtype, 1shg, SH3 polypeptide may be expressed, purified and their folding stability determined. Four candidates result equally or more stable polypeptides than the wildtype (mt12, mt13, mt17, mt18), three are less stable but folded (mt14, mt15, mt16) and one is partially unfolded (mt11). Thus, most mutants designed over the wildtype 1shg structure are found folded (mt12, mt13, mt14, mt15, mt16, mt17, mt18). As seen the CI method of the disclosure provides for a method from which an appropriate variant may be generated for different objectives.

All of the features and examples disclosed in this disclosure, including any accompanying claims, abstract and drawings, and/or all of the steps of any computer-implemented method or process, may be combined in any combination, except combinations where at least some of such features and/or steps are mutually exclusive. Each feature disclosed in this specification, including any accompanying claims, abstract and drawings, may be replaced by alternative features serving the same, equivalent or similar purpose, unless expressly stated otherwise. The scope of protection is defined by the claims.

## Claims

1. A computer-implemented method of assigning an identity of an amino acid residue to a reference position in a polypeptide backbone, the identity being determined by the amino acid residue side-chain;
the polypeptide backbone comprised within a macromolecular structure, the macromolecular structure comprising a plurality of elements, the elements comprising:
**an amino acid backbone in the reference position or an amino acid residue in the reference position;
**one or more neighbouring elements of a list consisting of: one or more further amino acid residues, one or more small molecules, one or more inorganic molecules, one or more nucleotides, at least a part of a lipid molecule, at least a part of a carbohydrate molecule, and combinations thereof; wherein a neighbouring element comprises at least one atom within a neighbouring distance from at least one atom of the amino acid backbone in the reference position or of the amino acid residue in the reference position;
the computer-implemented method comprising:
obtaining the reference position;
obtaining spatial coordinates of
at least one atom comprised in the amino acid backbone in the reference position or in the amino acid residue in the reference position; and
at least one atom per neighbouring element;
determining at least one reference multiplet of atoms, the reference multiplet consisting of one atom comprised in the amino acid backbone in the reference position or in the amino acid residue in the reference position and one atom of each of one or more neighbouring elements; wherein the reference multiplet of atoms is associated to a reference geometrical property; wherein the distance between the atoms of the reference multiplet comprised within each pair of two or more neighbouring elements is equal to or less than a neighbouring distance;
iteratively, per each one of the determined reference multiplet of atoms, finding, in a database, at least one matching multiplet of atoms;
wherein the atoms in each matching multiplet are ordered according to an ordered relationship between atoms;
wherein the database comprises
multiplets of atoms within elements deemed to interact in empirical macromolecular structures, and,
for each one of the multiplets:
an identity of each element comprising each one of the atoms,
and an associated geometrical property;
wherein the associated geometrical property of the at least one matching multiplet of atoms substantially equals the reference geometrical property;
determining the identity of the amino acid residue as the identity of an element deemed to interact comprising the atom of the at least one matching multiplet which corresponds, according to the ordered relationship, to the atom of the reference multiplet comprised in the amino acid backbone in the reference position or in the amino acid residue in the reference position;
and assigning the identity of the amino acid residue to the reference position in the polypeptide backbone.

2. The computer-implemented method of claim 1 wherein the at least one atom comprised in the polypeptide backbone is one of the alpha carbon atom or the C-beta atom and wherein the neighbouring element comprises at least one of the alpha carbon atom or the beta carbon atom within a neighbouring distance from the alpha carbon atom or the beta carbon atom of the polypeptide backbone respectively.

3. The computer-implemented method of claim 1 or claim 2 wherein the at least one matching multiplet of atoms comprises atoms of the same type as the type of the atoms of the reference multiplet.

4. The computer-implemented method of any one of claims 1 to 3 wherein finding the at least one matching multiplet of atoms comprises finding a plurality of matching multiplet of atoms which are comprised in the database.

5. The computer-implemented method of any one of claims 1 to 4 wherein
- the matching multiplet of atoms comprises at least one atom of an element with a predefined identity selected from a list of predefined identities and
- wherein the atom of the element with the predefined identity is comprised in the matching multiplet of atoms in a predefined position within the ordered relationship.

6. The computer-implemented method of any one of claims 1 to 5 wherein the identity of the assigned amino acid residue has different biochemical properties from the biochemical properties of the amino acid residue in the reference position, wherein the different biochemical properties may include one or more of charge, hydrophobicity and size, such that substituting the amino acid residue in the reference position with the assigned amino acid residue does not result in a conservative substitution.

7. The computer-implemented method of any one of claims 1 to 6 wherein the geometrical property comprises one or more distances between pairs of atoms, or angles between lines joining pairs of atoms in the multiplet, or dihedrals between planes comprising three or more of the atoms in the multiplet.

8. The computer-implemented method of claim 7 wherein the associated geometrical property of the at least one matching multiplet of atoms comprises one or more distances, between at least one pair of atoms comprised within a respective pair of elements that comprise the atoms of the at least one matching multiplet, which are equal to or less than the contacting distance.

9. The computer-implemented method of any one of claims 1 to 8 wherein the multiplet is a triplet of elements or a quartet of elements.

10. The computer-implemented method of any one of claims 1 to 9 wherein determining the identity of the amino acid residue comprises a scoring of elements deemed to interact, the elements deemed to interact comprising the atom of the at least one matching multiplet.

11. The computer-implemented method of any of claims 1 to 10 further comprising predicting a three-dimensional structure of a polypeptide comprising the polypeptide backbone with the assigned identity of the amino acid residue in the reference position.

12. The computer-implemented method of claim 11 further comprising estimating the stability of the polypeptide.

13. A computer program comprising instructions which, when the program is executed by a computer, cause the computer to carry out the computer-implemented method of any one of claims 1 to 12.

14. A macromolecular structure comprising a polypeptide comprising an amino acid residue in a reference position, the amino acid residue having an identity assigned by the computer-implemented method according to any one of claims 1 to 13.

15. A method to produce a macromolecular structure, the macromolecular structure according to claim 14.
